# EUROPEAN PATENT APPLICATION

(11) **EP 0 928 604 A1**
(43) Date of publication of application: **14.07.1999**
(21) Application number: 97310054.8
(22) Date of filing: 12.12.1997
(51) Int. Cl.: A61F 2/06, A61L 27/00, C08F 246/00

(54) **Stent**

(71) Applicant: BIOCOMPATIBLES LIMITED, Farnham, Surrey GU9 8QL (GB)
(72) Inventor: Woodroffe, Matthew, c/o Biocompatibles Ltd., Farnham, Surrey, GU9 8QL (GB); Brewer, Jason, c/o Biocompatibles Ltd,, Weydon Lane, Farnham, Surrey, GU9 8QL (GB); Russell, Jeremy, c/o Biocompatibles Ltd., Farnham, Surrey, GU9 8QL (GB); Moore, David,, Leicester LE9 7SA (GB)
(74) Representative: Haley, Stephen

(57) **Abstract**

A stent for implantation into a body passage comprising first and second sets of helical counter-rotating metallic filaments which are braided together to define a tubular stent body which is biased toward a first radially expanded configuration and which is compressible into a second radially compressed configuration, the filaments being encased in an elastomer having a hardness of up to 85 on the Shore A hardness scale.

## Description

The present invention relates to an implantable stent for transluminal implantation in a body cavity, such as peripheral and coronary blood vessels, but also for use in bile ducts, the oesophagus, urethras, ureters or ileums.

There are several designs of stents, which are usually permanently implantable devices, for transluminal insertion into blood vessels and other lumen to prevent or reverse occlusion thereof. There are three basic categories of device, namely heat-expandable devices, balloon-expandable devices and self-expanding devices. The present invention is concerned with self-expanding devices, that is which are inserted into the body lumen in a radially compressed condition and which are mechanically biased towards a radially expanded position. Upon being released in the cavity at the desired position, the stent expands radially exerting outwardly directed pressure upon the inner surface of the wall of the body lumen in which it is positioned.

One such expanding device which is commercially available is the so-called Wallstent. The device is described in WO-A-83/03752. It consists of two sets of counter-rotating helical filaments of metallic wire which are braided together in a one over/one under pattern.

There are difficulties with the braided stent of this type. One difficulty is that the change in axial length of the stent between the radially expanded and the radially compressed condition is generally high.

Another difficulty with braided stents in general is the tendency of the filaments at the end of the stent to unravel and splay outwards before or after deployment. This tendency makes the stent difficult to handle and the splayed ends can damage the inside wall of the body vessel in which the stent is deployed. In WO-A-83/03752, it is suggested that the filaments may be joined to one another at the end of the stent. However, as explained in a later specification by Wallsten et al in US-A-5,061,275, for stents with a high angle α between counter-rotating filaments, this rigidifies the ends of the prosthesis. This makes it difficult to compress the stent into its radially compressed condition. It is also said to accentuate the risk of penetration of the ends of the filaments through the wall of the lumen.

Another example of a braided stent is described in EP-A-0,183,372. The filaments in this publication are formed preferably of plastics material and the device generally has a warp thread extending parallel to the axis of the stent. The warp filament may be formed of shrinkable plastics to allow expansion of the stent insitu to its radially expanded condition.

According to a first aspect of the invention there is provided a stent for implantation into a body passage comprising first and second sets of helical counter-rotating metallic filaments which are braided together to define a tubular stent body which is biased toward a first radially expanded configuration and which is compressible into a second radially compressed configuration, the filaments being encased in an elastomer having a hardness of up to 85 on the Shore A hardness scale.

Preferably, the elastomer has a hardness up to 25 on the Shore A hardness scale. More preferably, the elastomer has a hardness up to 20 on the Shore A hardness scale.

The stent may be tapered in its axial direction.

The elastomer may be silicone rubber.

Encasing the stent in a relatively soft elastomer provides, contrary to expectations, encasement which is not readily punctured by the filaments at their ends, yet axial expansion upon radial compression is still significantly less than that for prior art stents as the filament's ends are held within the elastomer.

The first radially expanded diameter is the diameter adopted by the stent when no externally directed force is exerted upon it, that is when it expands in air. This diameter is somewhat greater than the internal diameter of the passage into which stent is to be implanted since this results in the stent exerting a continuous outwardly directed force on the internal wall of the body lumen in which it is located. In this fully unloaded configuration the angle α between filaments may be less than 90°. Generally it is less than 85°, preferably in the range 65-85°, most preferably in the range 70 to 80°.

Preferably the angle between the filaments at a crossover point at the mid point of the stent in situ when implanted in a body passage is in the range 60-90°, preferably in the range 65-75°.

The stent may be provided with a biocompatible coating, in order to minimise adverse interaction with the walls of the body vessel and/or with the liquid, typically blood, urea or bile flowing through the vessel. Examples of suitable coatings are described in our earlier applications numbers WO-A-93/01221, WO-A-94/16748, WO-A-94/16749 and WO-A-93/15775. Polymers as described in these specifications are haemo-compatible as well as generally biocompatible and, in addition, are lubricious.

It is important to ensure that the metallic surfaces of the stent are completely encased in order to minimise unfavourable interactions, for instance with blood, which might lead to thrombosis. If the stent encasing material is not biocompatible, then it will usually be necessary to provie a further outer coating. The thickness of the casing should, however, be as low as possible. A thickness of 0.2mm on each side of a 0.16mm diameter wire has been found to be a sufficient encasement for a urological stent of 9.3mm diameter without affecting compression characteristics.

It is preferred that the stent be formed from at least 4, more preferably at least 8 and most preferably at least 12 filaments in each direction. The number of filaments depends at least in part upon the diameter of each filament as well as the desired diameter and the desired size of the openings between the filaments of the stent in its radially expanded and contracted condition. The number of filaments and their diameter affects the flexibility of the stent in its radially contracted condition during delivery and it is preferred for the stent in that condition to be as flexible as possible. Generally the number of filaments in each direction is less than 32, more preferably from 24 downwards.

The filaments may be made from circular section wire. It may, alternatively be advantageous for rectangular section wire to be used. The use of flat (rectangular section wire) may provide optimum radial strength characteristics whilst minimising the overall thickness of the stent, especially at the crossover points, thereby minimising any interference of the liquid flow in the body passageway. The area of contact between wires at the crossover points is maximised by the use of flat wire which increases the amount of friction between the wires upon relative movement, for instance during any changes in radius. This should increase the resistance of the expanded stent to radial contraction in use although it may be disadvantageous to increase this area during delivery. The use of oval wire (with the smaller dimension being arranged substantially radially with respect to the stent axis) may provide a particularly advantageous combination of strength whilst minimising the contact area at crossover points.

The thickness of the filaments depends upon the desired final diameter (open diameter) of the stent. Wire having a diameter of 0.04 mm upwards, for instance up to 0.20 mm may be used. Wire with diameters at the lower end of the range would generally be used for making stents for use in small blood vessels, for instance in coronary arteries, where the diameters of the stents is generally in the range 0.5 mm up to 4.0 mm (fully radially expanded diameter). Larger stents may be used in peripheral blood vessels, aortic aneurisms or in stents for use in urological passageways, the oesophagus and in the bile duct, where the stent may have a diameter up to about 30 mm.

The length of the stent depends on the intended application of the stent. For instance in peripheral arteries the stent may have a length for instance, in the range 100 to 300 mm. For coronary arteries, the length may be in the range 10 to 50 mm. For male urethral stitcher relief the length may be in the range 50mm-300mm.

For most of the passageways, the diameter of the stents in the first radially expanded conformation is substantially constant along the length of the stent. The stent may flare or have a reduced diameter towards the end portion, in some instances. However, for an insertion into some body passages it is preferred for the diameter, that is the cross-sectional area, to vary along the length of the stent. For instance it may reduce migration of a device by providing it with a varying diameter along its length such that increased diameter sections and/or reduced diameter sections locate at and interact with, respectively, increased diameter body passageways (for instance openings into a higher volume organ) or reduced diameter sections, for instance at a sphincter. Such varying diameter portions may be provided by use of an appropriate braiding mandrel, or alternatively by a post-braiding heat treatment. One particular application of a varying diameter stent is a stent for use in urological passageways, for instance to overcome benign prostatic hyperplasia.

The filaments from which the braided stents are made are formed of a metal, for instance surgical steel, usually of a type having good elastic properties, for instance a high cobalt steel. These such materials give a stent having good self-expanding capability.

In addition to the self-expanding capability of the stent, it may be provided with a temperature dependent mechanical characteristic which allows a mechanical property of the stent to be changed by heating the stent from a temperature below transition temperature to above transition temperature. Thus some or all of the filaments may be formed from a shape memory alloy material such as nitinol. In such cases, in the stent prior to implantation, the stent is at a temperature below the transition temperature at which the metal changes from the martensitic structure to the austenitic structure. The filaments are adapted such that a transition from below the transition temperature to above the transition temperature will result in the stent either adopting a radially further expanded configuration or, preferably, retaining the same shape but having an increased resistance to radial collapsing under inwardly exerted pressure, due to the greater hardness of the metal at the higher temperature.

The alloy which is used in this aspect of the invention preferably has a transition temperature in the range 30 to 45°C, more preferably around body temperature. It can be heated either by contact with a heater or, where the transition temperature is about 35 to 40°C, by being implanted in the patient where it will warm up from room temperature to body temperature, i.e. to the transition temperature. Such alloys are known. Where such an alloy is used it may be used to form all or only some of the filaments of the stent.

The stent can be delivered using conventional delivery devices for self-expanding stents generally by percutaneous transluminal techniques.

The present invention is illustrated further in the accompanying figure in which:

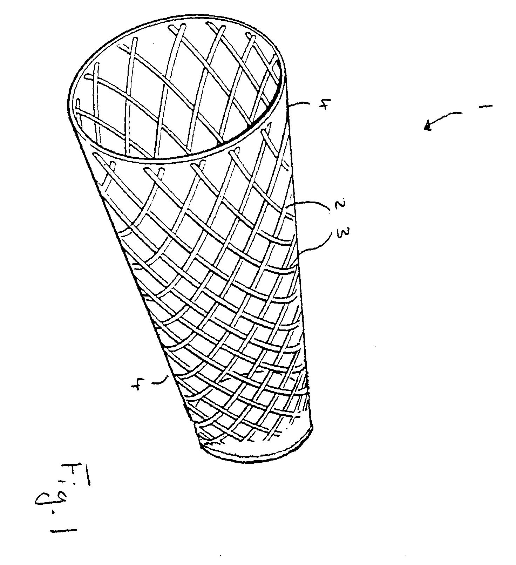

Figure 1 is a side view of a stent according to the present invention in relaxed, radially expanded condition.

As shown in figure 1, a stent 1 is formed of twelve wire filaments 2 arranged in right handed helices and twelve filaments 3 arranged in left handed helices. The filaments are braided in a one over/one under pattern. The angle α between the filaments in the radially expanded (relaxed, unloaded) condition is generally in the range 60-90, in this embodiment in the range 68-72°. Each filament, executes just over one complete turn (about 1¼ turns) within the length of the stent 1.

The stent 1 illustrated in figure 1 is, for instance, suitable for implanting in a urethra. The diameter is in the range 9 to 15mm and, in this example, tapers from 15mm at one end to 9mm at the other. The diameter of the stent in its axially compressed condition is generally at least ¹/₃ less than its expanded diameter. The wire used to form the filaments has a circular section and a diameter of 0.16mm. The wire is formed from a surgically implantable steel.

The stent 1 in its compressed condition has an angle a between the filaments which is reduced by 10 to 60% of the original angle. The stent 1 can be retained in its compressed condition either by exerting radial inwardly directed forces from the stent along its length, or by exerting axially outwardly directed forces at the ends of the stent.

The stent filaments 2,3 are encased in an elastomer 4, in this example silicon rubber, which has a Shore A hardness of up to 85, although preferably 20 or less.

As well as making it convenient to extend the stent 1, and stabilise it against flaring at the ends, the sealing of the ends of the filaments allows the stent 1 further to be axially compressed by exerting axially inwardly directed pressure against each end, so as to expand the radius of the stent, especially in its central portion, beyond the diameter. The stent 1 can thus be used to exert radially outwardly forces at a greater radial distance from the axis (than d₁) inside the blood vessel, for instance adding to or replacing the step of balloon dilatation prior to stent deployment. With the angle α being less than 90°, the use of the stent as a dilation device is convenient since a relatively large increase in diameter can be achieved with a relatively small axial reduction in length (as compared to a stent with a higher value of α).

A stent according to the invention can be formed as follows. Firstly, the filaments 2,3 are braided on a mandrel. Once braided, the filaments 2,3 are severed around the circumference located midway between two series of crossover points. With the filaments secured they can be removed from the mandrel.

The braiding may be formed such that there is an axial taper formed in the stent, alternatively this taper may be induced by heat treatment or during encasing in the elastomer as discussed below.

The filaments 2,3 are encased by forming an inner encasing layer 4 on the inner surface of the filaments 2, 3. This layer should be fairly thin, and should, preferably, be of a similar thickness or only a little thicker than the diameter of the filaments 2,3. The inner encasement may be formed so that the ends of the filaments 2,3 are still exposed. It may or may not be formed so as to seal all the gaps between filaments 2,3.

An outer encasing layer of silicone rubber, which may be of the same type as the inner layer is then applied. This may be applied with one end of the stent 1 being in compression so that a taper can be formed. Again it may or may not seal gaps between filaments 2,3. If necessary the ends of the stent 1 are then dipped in the same elastomer 4 to seal them and prevent the ends of the filaments 2,3 breaking through the encasing elastomer 4 in use.

## Claims

1. A stent for implantation into a body passage comprising first and second sets of helical counter-rotating metallic filaments which are braided together to define a tubular stent body which is biased toward a first radially expanded configuration and which is compressible into a second radially compressed configuration, the filaments being encased in an elastomer having a hardness of up to 85 on the Shore A hardness scale.

2. A stent according to claim 1, wherein the elastomer is a latex, a polyurethane or a silicone rubber.

3. A stent according to claim 1 or 2, wherein the encasing elastomer has a Shore A hardness of up to 25.

4. A stent according to claim 3, wherein the encasing elastomer has a Shore A hardness of up to 20.

5. A stent according to any of the preceding claims, in which the angle, α, is between filaments in the first configuration in the range 60° to 90°, preferably 65° to 85°, more preferably 70° to 80°.

6. A stent according to claim 5 in which the angle at which the filament ends are fixed is in the range α-5 to α.

7. A stent according to any preceding claim which has a polymeric biocompatible coating.

8. A stent according to claim 7, wherein the coating is a polymer having a zwitterionic pendant group.

9. A stent according to claim 8, wherein the podant group is a trimethyl ammonium ethyl phosphate ester group.

10. A stent according to any preceding claim which is encapsulated in a biocompatible bulk material.

11. A stent according to claim 10 wherein the stent is encapsulated in a silicone having a zwitterionic pendant group, more preferably a trimethylammonium etlyl phosphat ester group.

12. A stent according to any preceding claim in which the filaments are free to slide over each other at the crossover points.

13. A stent according to any preceding claim in which the filaments are formed of a shape memory alloy and in which the stent adopts the said first radially expanded configuration below the transition temperature and, when the stent is treated to a temperature above the transition temperature the stent tends to adopt a maximally radially expanded configuration in which the diameter is increased and/or the resistance of the stent to radial compression is increased.

14. A stent according to any of the preceding claims, wherein the elastomer is continuous along the entire stent to provide a totally encased tube.
